# EUROPEAN PATENT SPECIFICATION

(11) **EP 2 652 504 B1**
(45) Date of publication and mention of the grant of the patent: **12.11.2014**
(21) Application number: 11794755.6
(22) Date of filing: 14.12.2011
(51) Int. Cl.: G01N 33/574

(54) **METHOD FOR DISCRIMINATING BETWEEN EARLY AND ADVANCED STAGE LUNG CARCINOMA**
VERFAHREN ZUR UNTERSCHEIDUNG ZWISCHEN LUNGENKARZINOM IM FRÜHEN UND IM FORTGESCHRITTENEN STADIUM
PROCÉDÉ DE DISCRIMINATION ENTRE UN CARCINOME PULMONAIRE EN STADE PRÉCOCE ET UN CARCINOME PULMONAIRE EN STADE AVANCÉ

(30) Priority: 14.12.2010 EP 10194916
(43) Date of publication of application: 23.10.2013
(73) Proprietor: Aposcience AG, 1010 Wien (AT)
(72) Inventor: ANKERSMIT, Hendrik Jan, A-1160 Vienna (AT)
(74) Representative: KLIMENT & HENHAPEL
(86) International application number: PCT/EP2011/072703
(87) International publication number: WO 2012/080303

(56) References cited:
- MALUSECKA E ET AL: "EXPRESSION OF HEAT SHOCK PROTEINS HSP70 AND HSP27 IN PRIMARY NON-SMALL CELL LUNG CARCINOMAS: AN IMMUNOHISTOCHEMICAL STUDY", ANTICANCER RESEARCH, INTERNATIONAL INSTITUTE OF ANTICANCER RESEARCH, GR, vol. 21, no. 2A, 1 January 2001 (2001-01-01), pages 1015-1022, XP009026698, ISSN: 0250-7005
- VARGAS S O ET AL: "Estrogen-receptor-related protein p29 in primary nonsmall cell lung carcinoma: pathologic and prognostic correlations.", CANCER 15 APR 1998 LNKD- PUBMED:9554526, vol. 82, no. 8, 15 April 1998 (1998-04-15) , pages 1495-1500, XP002629126, ISSN: 0008-543X
- MALUSECKA EWA ET AL: "Stress proteins HSP27 and HSP70i predict survival in non-small cell lung carcinoma", ANTICANCER RESEARCH, INTERNATIONAL INSTITUTE OF ANTICANCER RESEARCH, GR, vol. 28, no. 1B, 1 January 2008 (2008-01-01), pages 501-506, XP008111747, ISSN: 0250-7005
- SEIWERT TANGUY Y ET AL: "HSP27 - A novel prognostic marker and therapeutic target for non-small cell lung cancer.", PROCEEDINGS OF THE AMERICAN ASSOCIATION FOR CANCER RESEARCH ANNUAL MEETING, vol. 47, April 2006 (2006-04), page 303, XP002629127, & 97TH ANNUAL MEETING OF THE AMERICAN-ASSOCIATION-FOR-CANCER-RESEARCH (AACR); WASHINGTON, DC, USA; APRIL 01 -05, 2006 ISSN: 0197-016X
- HUANG QI ET AL: "Expression of heat shock protein 70 and 27 in non-small cell lung cancer and its clinical significance.", JOURNAL OF HUAZHONG UNIVERSITY OF SCIENCE AND TECHNOLOGY. MEDICAL SCIENCES = HUA ZHONG KE JI DA XUE XUE BAO. YI XUE YING DE WEN BAN = HUAZHONG KEJI DAXUE XUEBAO. YIXUE YINGDEWEN BAN 2005 LNKD- PUBMED:16696328, vol. 25, no. 6, 2005, pages 693-695, XP002629128, ISSN: 1672-0733
- DENG BIN ET AL: "Stage-specific proteins expression of human squamous lung carcinomas by a proteomics approach", MOLECULAR & CELLULAR PROTEOMICS; 3RD ANNUAL WORLD CONGRESS OF THE HUMAN-PROTEOME-ORGANISATION (HUPO); BEIJING, PEOPLES R CHINA; OCTOBER 25 -28, 2004, AMERICAN SOCIETY FOR BIOCHEMISTRY AND MOLECULAR BIOLOGY, INC, US, vol. 3, no. 10 supplement S, 28 October 2004 (2004-10-28), page S57, XP008134529, ISSN: 1535-9476
- DENG BIN ET AL: "Proteomics analysis of stage-specific proteins expressed in human squamous cell lung carcinoma tissues.", CANCER BIOMARKERS : SECTION A OF DISEASE MARKERS 2005 LNKD- PUBMED:17192052, vol. 1, no. 6, 2005, pages 279-286, XP008134618, ISSN: 1574-0153

## Description

The present invention relates to a method for discriminating between early and advanced stage lung carcinoma in an individual suffering from lung carcinoma.

Lung cancer is the most common cancer in terms of mortality worldwide. The incidence rate is estimated at 1.2 million, the mortality-rate at 1.1 million cases per year worldwide. It is by far the most common cancer of men and the second most malignant tumor of women after breast cancer.

The prognosis of lung cancer has improved little in the last 20 years and a large proportion of newly discovered cancer cases still takes a fatal course. The 5-year survival is currently 10% in men and 14% in women, but heavily depends on the time of diagnosis. 5-year survival rates of 29-43% in local stage face therefore survival rates of 11-16% in regional stage and 1-2% in distant stage.

The increase in lung cancer at remaining poor prognosis shows the importance of appropriate approaches to primary prevention, early detection and diagnosis. In no other form of cancer as many deaths as in lung cancer could be prevented by effective prevention.

Patients suffering from lung cancer can be treated in various ways including surgery, radiotherapy, chemotherapy and combinations thereof. Surgery is the primary treatment for patients with early stage cancer who are in good general health. The goal of surgery is to totally eliminate all the cancer cells and thereby provide a cure. Even if a cancer recurs after an attempt to remove it, the recurrent cancer often can be removed in a second operation. Radiotherapy can be applied as a primary treatment, before surgery to shrink the cancer, after surgery to eliminate any cancer cells that remain in the treated area or have spread to other areas of the body. Chemotherapy involves drugs that are toxic to cancer cells. The drugs are usually given by direct injection into a vein or through a catheter placed in a large vein. Often given after surgery to get rid of small groups of cancer cells that may remain, chemotherapy also may slow cancer growth and relieve symptoms in patients who cannot have surgery.

In order to apply the optimal therapy to the patients suffering from lung cancer it is crucial to know to which stage the disease already progressed. If a patient suffers from early stage lung carcinoma surgery optionally combined with other therapies is the method of choice to remove the cancer from the body of said patient. If the patient is diagnosed to have lung carcinoma in an advanced stage surgery will not be the primary method to treat said patient. In such a case other methods have to be applied.

Failing early clinical symptoms, early detection of lung cancer can be achieved only by screening tests. Technologies such as spiral computed tomography (CT) and autofluorescence bronchoscopy can detect lung cancers down to the submillimeter range. However, the wide variations in lung cancer risk, even among longterm smokers, makes these sensitive technologies neither practical nor cost-effective as screening tools in the general population. Application of a filter to identify smokers at the highest risk for lung cancer may improve the positive predictive value of these screening tools. A blood based biomarker is an attractive filter because blood is easily accessible and measurements may be repeated over time.

Several studies have shown that the expression of different biomarkers is frequent in cell lines of lung cancer and many authors have considered the presence of these markers in blood as an index of the extension of disease, prognosis and response to therapy. Some tumor markers (NSE, Chromogranine, CEA, TPA and CYFRA 21-1) have been evaluated in many patients with small cell lung cancer (SCLC) as well as with non-small cell lung carcinoma (NSCLC). Qi et al (2005) disclose HSP70 as lung cancer stage specific biomarker. However, the diagnosis and follow-up of this pathology are not yet satisfactory.

It is an object of the present invention to provide methods for discriminating between various stages of lung cancer.

The present invention relates to a method for discriminating between early and advanced stage lung carcinoma in an individual suffering from lung carcinoma as claimed.

Heat Shock Proteins, categorized in families by their molecular weight, are highly conserved molecules whose expression is increased in most stressed cells. Indeed, they are implicated in several functions related to cell viability and cell protection. Among other functions, HSP are also known to be required for cell division.

The expression pattern in cancer is frequently different from that observed in normal cells: most often some stress-inducible HSPs are constitutively and highly expressed. Increased expression of some HSP has been documented in several human cancers, including lung cancer.

It turned out that the determination of HSP27 in a sample obtainable from a patient suffering from lung carcinoma allows to differentiate between the various stages of said disease allowing the physician to apply the best method for treating the patient. The level of HSP27 in patients suffering from advanced stage lung carcinoma is significantly higher than in patients suffering from early stage lung carcinoma. The latter group of patients shows a significantly higher level of HSP27 than healthy individuals.

It is important for the physician to know whether a patient suffers from early or from advanced stage lung carcinoma. As a rule patients suffering from early stage lung carcinoma can be treated by other methods than patients suffering from advanced stage lung carcinoma. Usually, patients suffering from early stage lung carcinoma can still be treated with surgical methods whereas patients suffering from advanced stage lung carcinoma are treated by other methods.

"Control individuals with early stage lung carcinoma" and "control individuals with advanced stage lung carcinoma" refer to a group of individuals which are diagnosed to suffer from early and advanced stage lung carcinoma. These groups of individuals comprise at least 5, preferably at least 10, more preferably at least 20, even more preferably at least 30, individuals all of them diagnosed to suffer from lung cancer at the respective stages.

Lung cancer is often suspected after an abnormal spot is found on a chest x-ray done to evaluate a cough or chest pain.

When lung cancer is suspected, a thorough history and physical exam will be performed first. This is done to evaluate symptoms and risk factors for lung cancer and to look for any physical signs suggestive of lung cancer. These can include changes in the character of the cough, abnormal lung sounds, haemoptysis, dyspnoea or enlarged lymph nodes.

A chest x-ray is usually the first test performed to evaluate any concerns based on a careful history and physical exam. This may show a mass in the lungs or enlarged lymph nodes. Sometimes the chest x-ray is normal and further tests are needed to look for a suspected lung cancer. Even if a mass is found, these are not always cancerous and further studies are needed.

A CT scan is frequently the second step either to follow up on an abnormal chest x-ray finding, or to evaluate troublesome symptoms in those with a normal chest x-ray.

CT/PET fusion imaging, combines the technology of CT scan with the technology of PET (positive emission tomography) scan. PET scans involve injecting a sugar-based radiopharmaceutical, which travels through the body and collects in organs and tissues, which are metabolically active. The PET scan is used to detect cancer cells in the body and the CT scan provides detailed images that can determine the location and size of the cancer. This type of scan differs from the others in that it defines tumors that are actively growing.

After a lung cancer is suspected based on imaging, a sample of tissue is required to confirm the diagnosis and determine the type of cancer. Sputum cytology is the easiest way to do this, but its use is limited to those tumors that extend into the airways. Sputum cytology is not always accurate and can miss some cancer cells.

In case of central located processes diagnosis and histology can be ascertained by bronchoscopy, biopsy of endobrochial lesions, bronchoalveolar lavage or transbronchial needle biopsy.

If the tumor cannot be reached by bronchoscopy or similar procedures, fine needle aspiration (FNA) or video assisted thoracoscopy (VATS) can be performed. Thereby a hollow needle is inserted through the chest wall (FNA), respectively a thoracoscope is inserted through small incisions, to take a sample of the tumor.

Once a lung cancer diagnosis is made, the oncology team determines if the patient is a candidate for surgery by reviewing the imaging studies (e.g., x-ray, CT scan, bone scan) to rule out distant metastasis.

If there is no evidence of metastasis, the patient may then undergo mediastinoscopy, a surgical inspection of the mediastinum.

There are several lung cancer types, and it is important to determine the form of lung cancer in order to choose the best treatment options. The classification is done by a pathologist by light microscopic criteria according to the classification as proposed by the World Health Organization (WHO).

The treatment choices, as well as prognosis for a lung cancer, can vary widely depending on both the particular cancer type and the stage at which it is diagnosed.

"Reference range" as used herein is the range of HSP27 levels within which at least 80%; preferably at least 90%, more preferably at least 95%, even more preferably at least 99%, in particular 100%, of the HSP27 levels determined in the aforementioned control individuals can be found. The "reference range" comprises a minimal and a maximal level of HSP27. These levels define the upper and lower limits of the "reference range".

As used herein "below the reference range" means that the level determined is below or lower than the "reference range" as a whole. In other words the determined level is below or lower than the minimal or lower level of HSP27 of the "reference range".

As used herein "above the reference range" means that the level determined is above or higher than the "reference range" as a whole. In other words the determined level is above or higher than the maximal or upper level of HSP27 of the "reference range".

"Within the reference range" means that the HSP27 levels determined according to the present invention are in between the reference range as defined above.

In an alternative method it is also possible to define cut-off levels. Such cut-off levels serve as a limit to indicate whether a patient having defined HSP27 levels over or below said cut-off values suffer from early or advanced stage lung carcinoma. Therefore the present disclosure relates also to a method for discriminating between early and advanced stage lung carcinoma in an individual suffering from lung carcinoma comprising the steps of
a) providing a sample of an individual suffering from lung carcinoma,
b) determining the level of heat shock protein 27 (HSP27) in said sample,
c) comparing the determined level of HSP27 in said sample to cut-off levels of HSP27 in healthy individuals (i.e. individuals which do not suffer from a cancer or any other disease associated with an increased expression rate of HSP27) and/or individuals suffering from early and/or advanced stage lung carcinoma, said cut-off levels distinguishing between early and advanced stage lung carcinoma,
d) diagnosing early stage lung carcinoma in said individual when the level of HSP27 in sample a) is at least 5%, preferably at least 10%, more preferably at least 20%, below the cut-off value for suffering from advanced stage lung carcinoma and/or above the cut-off value of healthy individuals, and diagnosing advanced stage lung carcinoma in said individual when the level of HSP27 in sample a) is at least 5%, preferably at least 10%, more preferably at least 20%, above the cut-off value for suffering from early stage lung carcinoma.

These cut-off levels may be determined by determining the average or median HSP27 level in at least 5, preferably at least 10, more preferably at least 20, even more preferably at least 30, individuals all of them diagnosed to suffer from lung cancer at the respective stages or healthy individuals. The cut-off for diagnosing early stage lung carcinoma may the lowest average or median HSP27 level determined in control individuals suffering from advanced stage lung carcinoma as defined above or the highest average or median HSP27 level found in a number of healthy individuals. The cut-off for diagnosing advanced stage lung carcinoma may the highest average or median HSP27 level determined in control individuals suffering from early stage lung carcinoma as defined above.

According to the present invention the early stage lung carcinoma is stage Ia to IIb and advanced stage lung carcinoma is stage IIIa to IV as defined by the International Association for the Study of Lung Cancer (IASCL).

Definition of the stage of lung carcinoma is essential for deciding which type of treatment has to be applied. Thus has led to the development of a universally accepted stage classification system for most tumors. The Union Internationale Contre le Cancer (UICC) and the American Joint Committee on Cancer (AJCC) serve as the official bodies that define, periodically review, and refine the stage classification systems.

In 1998, the International Association for the Study of Lung Cancer (IASLC) created an International Staging Commitee (ISC) of multidisciplinary members and collected 68,463 patients with nonsmall lung cancer and 13,032 patients with small lung cancer, registered or diagnosed from 1990 to 2000, whose record had adequate information for analyzing the tumor, node, metastasis (TNM) classification. The T, N, M descriptors were analyzed and recommendations for changes in the seventh edition of the TNM classification were proposed based on differences in survival (Goldstraw et al., J Thorac Oncol. Aug 2007;2(8):706-714).

### UICC TNM Classification of Malignant Tumors - 7th edition (UICC (International Union Against Cancer) TNM Classification of Malignant Tumors - 7th edition. Sobin LH, Gospodarowicz M, Wittekind C ISBN: 978-1-4443-3241-4, Dec 2009)

| | | | |
|---|---|---|---|
| Occult carcinoma | TX | N0 | M0 |
| Stage 0 | Tis | N0 | M0 |
| Stage IA | T1a, b | N0 | M0 |
| Stage IB | T2a | N0 | M0 |
| Stage IIA | T1a, T1b, T2a | N1 | M0 |
| | T2b | N0 | M0 |
| Stage IIB | T2b | N1 | M0 |
| | T3 | N0 | M0 |
| Stage IIIA | T1a, T1b, T2a, T2b | N2 | M0 |
| | T3 | N1, N2 | M0 |
| | T4 | N0, N1 | M0 |
| Stage IIIB | T4 | N2 | M0 |
| | any T | N3 | M0 |
| Stage IV | any T | any N | any M |

### T (Primary Tumor)

| | |
|---|---|
| TX | Primary tumor cannot be assessed, or tumor proven by the presence of malignant cells in sputum or bronchial washings but not visualized by imaging or bronchoscopy |
| T0 | No evidence of primary tumor |
| Tis | Carcinoma in situ |
| T1 | Tumor less than or equal to 3 cm in greatest dimension, surrounded by lung or visceral pleura, without bronchoscopic evidence of invasion more proximal than the lobar bronchus (i.e., not in the main bronchus) |
| T1a | Tumor less than or equal to 2 cm in greatest dimension |
| T1b | Tumor > 2 cm but less than or equal to 3 cm in greatest dimension |
| T2 | Tumor > 3 cm but less than or equal to 7 cm or tumor with any of the following features: Involves main bronchus, greater than or equal to 2 cm distal to the carina; Invades visceral pleura; Associated with atelectasis or obstructive pneumonitis that extends to the hilar region but does not involve the entire lung |
| T2a | Tumor > 3 cm but less than or equal to 5 cm in greatest dimension |
| T2b | Tumor > 5 cm but less than or equal to 7 cm in greatest |
| | dimension |
| T3 | Tumor > 7 cm or one that directly invades any of the following: chest wall (including superior sulcus tumors), diaphragm, phrenic nerve, mediastinal pleura, parietal pericardium; or tumor in the main bronchus (< 2 cm distal to the carina but without involvement of the carina; or associated atelectasis or obstructive pneumonitis of the entire lung or separate tumor nodule(s) in the same lobe) |
| T4 | Tumor of any size that invades any of the following: mediastinum, heart, great vessels, trachea, recurrent laryngeal nerve, esophagus, vertebral body, carina, separate tumor nodule(s) in a different ipsilateral lobe |

### N (Regional Lymph Nodes)

| | |
|---|---|
| NX | Regional lymph nodes cannot be assessed |
| N0 | No regional lymph node metastasis |
| N1 | Metastasis to ipsilateral peribronchial and/or ipsilateral hilar lymph nodes and intrapulmonary nodes involved by direct extension of the primary tumor |
| N2 | Metastasis to ipsilateral mediastinal and/or subcarinal lymph node(s) |
| N3 | Metastasis to contralateral mediastinal, contralateral hilar, ipsilateral or contralateral scalene, or supraclavicular lymph node(s) |

### M (Distant Metastasis)

| MX | Presence of distant metastasis cannot be assessed |
|---|---|
| M0 | No distant metastasis |
| M1 | Distant metastasis |
| M1a | Separate tumour nodule(s) in a contralateral lobe; pleural nodules or malignant pleural or pericardial effusion |
| M1b | Distant metastasis |

Before treatment recommendations can be made for a given patient, the size of the tumor, lymph-node status, and possible presence of metastases must be ascertained. Lung cancer often spreads to the draining nodes in the hilum and mediastinum. Mediastinal involvement (N2) places the patient in a higher stage and may render the tumor unresectable, if it is close to vital mediastinal structures.

Clinical staging is based on the size of the lymph node as determined by CT. Until fairly recently, the use of a cutoff for normal nodes of 10 to 15 mm provided sensitivities and specificities for detecting nodal metastases of only 40 to 70 percent, but this approach was the only noninvasive option. Even small, peripheral T1 lesions, often considered to be associated with a low risk of mediastinal spread, frequently involve these nodes.

Positron-emission tomography (PET) has emerged as an important noninvasive test for mediastinal assessment. This approach operates on the principle that tumors cause increased uptake of radiolabeled glucose that can be imaged. Retrospective data from several trials suggest a sensitivity and specificity of 85 percent and 88 percent, respectively, for the mediastinal staging of lung cancer with the use of PET and the results of a prospective study support these findings.

The combination of PET and CT appears to have an even greater sensitivity and specificity than the use of either method alone and the use of both should be strongly considered as part of the preoperative evaluation.

The gold standard for mediastinal evaluation is lymph-node biopsy by means of bronchoscopy or, if needed, the more invasive mediastinoscopy. To distinguish potentially curable from incurable disease, radiologic studies that indicate the presence of mediastinal disease should be followed by biopsy of identified nodes before a tumor is deemed to be unresectable. However, with the method of the present invention no biopsy needs to be performed. Furthermore, the method of the present invention gives reliable data in a reduced time frame.

Complete surgical resection is considered the treatment of choice for individuals with stage I-II cell lung carcinoma. Surgery should include resection (pneumonectomy or lobectomy) and mediastinal-node mapping. Complete lymph-node dissection should be performed if the tumor is resectable and mediastinal nodes are involved.

Stage III tumors or those invading vital structures are often described as either nonresectable or marginally resectable. For many years, the mainstay of treatment for these tumors was radiotherapy (total dose, 60 Gy). The risk of local recurrence was diminished, but the rate of long-term survival was still poor (5 percent). The results of several phase 2 studies provided preliminary support for the addition of chemotherapy to radiotherapy, and a landmark trial of this combined approach, demonstrated increased rates of three-year survival (23 percent as compared with 11 percent) and long-term survival.

Currently, virtually no patient with disease as advanced as stage IIIB or IV will be cured. Although chemotherapy is the backbone of treatment for metastatic disease, response rates are low, and survival times poor. In the past, many patients with advanced lung carcinoma received no therapy, since the toxicity of therapy was thought to outweigh the benefits. It is now clear, however, that treatment can be beneficial. Several meta-analyses have reported moderate gains in survival when chemotherapy is used, as compared with the best supportive care. Increases in median survival appear to be in the range of two to four months, and increases in the one-year survival rate appear to range from 10 to 20 percent.

According to a preferred embodiment of the present invention the sample is a blood sample, preferably a serum or plasma sample.

It turned out that the HSP27 levels can be determined in a blood sample or a fraction thereof. This is particularly advantageous because it allows a rapid and reliable diagnosis.

Methods for determining the level of HSP27 in a sample are well known in the art. In a preferred embodiment of the present invention the level of HSP27 is determined by an immunoassay, preferably by a competitive or a non-competitive immunoassay, more preferably by a Western-blot, an enzyme-linked immunosorbent assay (ELISA), lateral flow immunoassay or a radioimmunoassay (RIA).

The aforementioned assays involve molecules which are able to specifically bind to HSP27. Such molecules can be, for instance, antibodies or fragments thereof, which are still able to bind to HSP27.

The term "antibody", as used herein, refers to monoclonal and polyclonal antibodies or fragments thereof capable to bind to an antigen. Other antibodies and antibody fragments, such as recombinant antibodies, chimeric antibodies, humanized antibodies, antibody fragments such as Fab or Fv fragments, as well as fragments selected by screening phage display libraries, and the like are also useful in the methods described herein.

Methods for preparation of monoclonal as well as polyclonal antibodies are well established (Harlow E. et ah, 1988. Antibodies. New York: Cold Spring Harbour Laboratory). Polyclonal antibodies are raised in various species including but not limited to mouse, rat, rabbit, goat, sheep, donkey, camel and horse, using standard immunization and bleeding procedures. Animal bleeds with high titres are fractionated by routine selective salt-out procedures, such as precipitation with ammonium sulphate and specific immunoglobulin fractions being separated by successive affinity chromatography on Protein-A-Sepharose and leptin- Sepharose columns, according to standard methods. The purified polyclonal as well as monoclonal antibodies are then characterised for specificity. Such characterization is performed by standard methods using proteins labeled with a tracer such as a radioisotope or biotin in competition with increasing levels of unlabeled potential cross-reactants for antibody binding. Binding studies are further evaluated by other standard methods such as the well-established sodium dodecyl sulphate-polyacrylamide gel electrophoresis (SDS-PAGE) and Western immunoblot methods under reducing and non-reducing conditions.

Monoclonal antibodies are prepared according to well established standard laboratory procedures. This technique is performed by removing spleen cells from immunized animals and immortalizing the antibody producing cells by fusion with myeloma cells or by Epstein-Barr virus transformation, and then screening for clones expressing the desired antibody, although other techniques known in the art are also used. Antibodies are also produced by other approaches known to those skilled in the art, including but not limited to immunization with specific DNA.

Preferably used immunoassays are based on techniques which use capture antibodies, which are able to specifically bind to an antigen of interest, or antigens bound on a solid support. The capture antibody is coupled with or linked to various solid phase supports using standard non-covalent or covalent binding methods, depending on the required analytical and/or solid-phase separation requirements. The solid-support is in the form of test tubes, beads, microparticles, filter paper, membranes, glass filters, magnetic particles, glass or silicon chips or other materials and approaches known to those skilled in the art. The use of microparticles, particularly magnetizable particles, that have been directly coated with the antibody (magnetic particles-capture antibody) or particles that have been labelled with a universal binder (e.g., avidin or anti-species antibody) is useful for significantly shortening the assay incubation time. These along with other alternative approaches known in the art allow for assay completion within minutes without limiting the required sensitivity.

The detection antibody, which is able to specifically bind to the antigen of interest, used for detection of the antigen is either directly coupled with a reporter molecule, or detected indirectly by a secondary detection system. The latter is based on several different principles known in the art, including antibody recognition by a labelled anti-species antibody and other forms of immunological or non-immunological bridging and signal amplification detection systems (e.g., the biotin-streptavidin technology). The signal amplification approach is used to significantly increase the assay sensitivity and low level reproducibility and performance. The label used for direct or indirect antibody coupling is any detectable reporter molecule. Examples of suitable labels are those widely used in the field of immunological and non-immunological detection systems, such as fluorophores, luminescent labels, metal complexes and radioactive labels, as well as moieties that could be detected by other suitable reagents such as enzymes, or various combinations of direct or indirect labels such as enzymes with luminogenic substrates.

The reference level of HSP27 in control individuals with an early stage lung carcinoma amounts preferably to 3000 to 4400 pg/ml, preferably 3500 to 4200 pg/ml.

The reference level of HSP27 in control individuals with an advanced stage lung carcinoma amounts preferably to at least 4600 pg/ml, preferably 4600 to 6000 pg/ml, more preferably 5000 to 5600 pg/ml.

The reference level of HSP27 in healthy individuals amounts preferably to a maximum of 2500 pg/ml, preferably 500 to 2500 pg/ml, more preferably 1000 to 2100 pg/ml.

Of course these values may vary depending on the method used to determine the HSP27 levels.

HSP27 levels may also be used to monitor the progress of a treatment. The data presented herein allow also to monitor the success of the treatment of lung cancer. It is known that the level of HSP27 in healthy individuals is lower than in patients suffering from lung carcinoma. A method for monitoring the progress of a treatment may comprise the determination of the HSP27 at pre-defined time points during the treatment. The lowering of the HSP27 levels indicates that the treatment is successful or has advantageous effects on the health of the individual to be treated.

The present invention is further illustrated by the following figures and example.
Fig. 1 shows the determination of HSP27 in serum samples of patients.
Fig. 2 shows the determination of HSP70 in serum samples of patients.
Fig. 3 shows an univariate logistic regression model.

### EXAMPLE:

### Material and Methods

A total number of 187 patients and controls were included in this case control study.

Healthy volunteers without any clinical signs of lung carcinoma (n=36), patients with carcinoma diagnosed at an early stage (n=63) and patients with carcinoma diagnosed at an advanced stage (n=99) were evaluated in three study groups.

The classification early respectively advanced stage was based upon the histological statement and complies with the IASCL lung cancer staging project (Tsim S, et al. Respir Med. 104 (2010):1767-1774 ). Early stage includes the IASCL stages Ia to IIb, advanced stage includes stages IIIa to IV.

Patient characteristics are depicted in Table 1:

| | **Healthy** | **Stage Ia - IIb** | **Stage IIIa - IV** |
|---|---|---|---|
| N | 36 | 63 | 99 |
| *Male*/*Female* | 13/23 | 37/26 | 57/42 |
| *Age* | 57.84 | 64.20 | 61.87 |
| *Smoking History* | | | |
| *Never Smoker* | 66.7% | 15.8% | 9.9% |
| *Smoker* | 33.30 | 84.2% | 90.1% |
| *Histology* | | | |
| *Adenocarcinoma* | | 61.9% | 72.5% |
| *Squamous cell carcinoma* | | 25.4% | 26.4% |
| *Large cell carcinoma* | | 6.3% | 1,1% |
| *Neuroendocrine carcinoma* | | 6.3% | 0.0% |

Blood samples were collected at the time of first admission to the present study. Serum was acquired after centrifugation and aliquots were kept frozen at -80°C until further testing.

### Determination of Heat Shock Protein 27

Serum levels of HSP27 were determined using adapted enzyme-linked immunosorbent assay (ELISA) kits (Duoset IC; R&D Systems, USA).

Ninety-six-well microtiter plates were coated overnight at 4°C with capture antibody at a concentration of 1ug/ml. After blocking of plates, serum samples and standard protein in different concentrations were added to the wells. After a washing step, a biotin-labelled antibody was added to each well and incubated for 2 hours. Plates were washed and Streptavidin-HRP was added. Color reaction was achieved using tetramethylbenzidine (TMB; Sigma, USA) and was stopped by an acid stop solution. Optical density was measured at 450 nm on an ELISA reader.

### Determination of Heat Shock Protein 70

Serum levels of HSP70 were determined using adapted enzyme-linked immunosorbent assay (ELISA) kits (Duoset IC; R&D Systems, USA).

Ninety-six-well microtiter plates were coated overnight at 4°C with capture antibody at a concentration of 2ug/ml. After blocking of plates, serum samples and standard protein in different concentrations were added to the wells. After a washing step, a biotin-labelled antibody was added to each well and incubated for 2 hours. Plates were washed and Streptavidin-HRP was added. Color reaction was achieved using tetramethylbenzidine (TMB; Sigma, USA) and was stopped by an acid stop solution. Optical density was measured at 450 nm on an ELISA reader.

### Results

### Heat Shock Protein 27

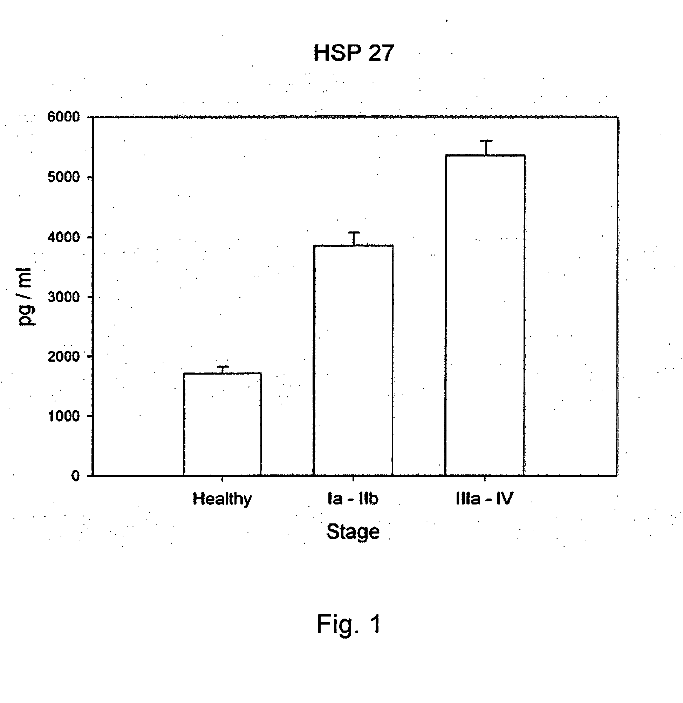

Serum levels of HSP27 were 1751 pg/ml [±113.31] (mean [±SEM]) in healthy controls, 3856 pg/ml [±215.57] in patients with carcinoma diagnosed at an early stage as defined above and 5350 pg/ml [±254.23] in patients with carcinoma diagnosed at an advanced stage as defined above. Statistically significant differences were found between all three groups (in each case p<0.0.01) (Fig. 1).

### Heat Shock Protein 70

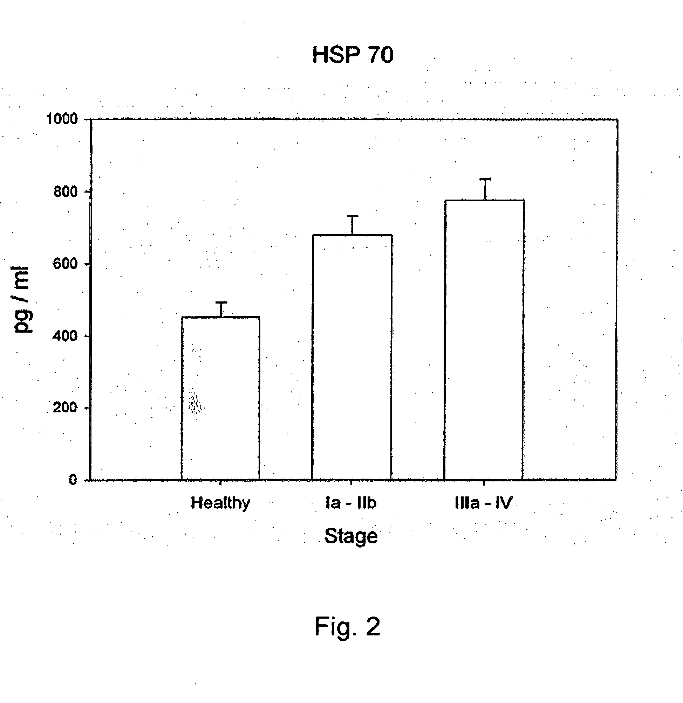

Mean serum levels of HSP70 were 451 pg/ml [±41.19] (mean [±SEM]) in healthy controls, 679 pg/ml [±53.93] in patients with carcinoma diagnosed at an early stage and 777 pg/ml [±57.93] in patients with carcinoma diagnosed at an advanced stage. Statistically significant differences were found between healthy controls and the two cancer groups (p<0.001), but not between the two groups with carcinoma diagnosed at different stages (Fig. 2).

### Regression Models

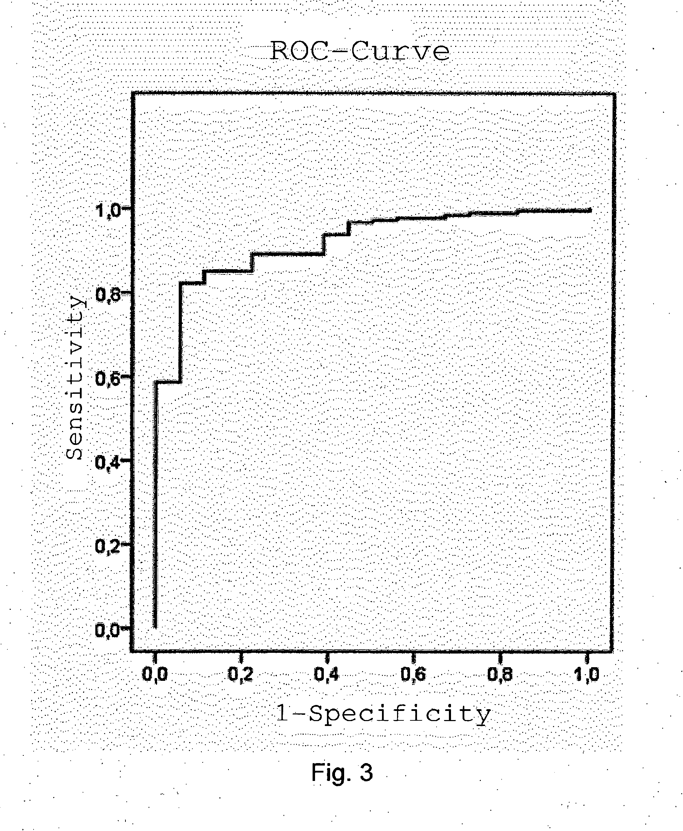

In univariate logistic regression model including only healthy volunteers and patients with diagnosed carcinoma of the lung, HSP27 had an area under the curve (AUC) in the receiver operating characteristics (ROC) curve of 0.920 (±0.027; p<0.001) (Fig. 3).

### Discussion

Heat Shock Proteins play key roles in cell protection and cell viability. They are also known to be required for cell division and may contribute to cell proliferation.

Apart from their protective roles in the cytosol, HSPs have been found to play an important role in the stimulation of the immune system when located in the extracellular space or on the plasma membrane. In vitro, members of the HSP70 and HSP90 families have been detected in the medium of antigen-presenting cells (APCs). Although the immunological role of extracellular and membrane-bound HSPs appears apparent, the mechanism of transport to the plasma membrane, the membrane anchorage, and the export remains enigmatic.

Several mechanisms may account for the cellular release of HSPs, including exportation into the medium in concert with other proteins possessing transmembrane domains that fulfil shuttle functions, through direct interaction with lipid components, through flip-flop mechanism after binding to phosphatidylserine or by necrosis.

HSPs like HSP70, HSP90, and Gp96 have been found in the extracellular medium.

In the present example an increase of HSP27 in serum samples taken from the peripheral blood flow of patients with a lung cancer could be shown. Furthermore these results demonstrate a continuous increase of serum HSP27 concentrations with disease severity.

Therefore serum contents of HSP27 showed diagnostic potential to determine the occurrence of lung cancer and may serve as a marker for diagnosis and prediction of disease progression. Furthermore the level of HSP27 in serum of patients with lung cancer may provide a basis for therapy decision, especially when the question arises if curative surgery is possible.

## Claims

1. Method for discriminating between early and advanced stage lung carcinoma in an individual suffering from lung carcinoma comprising the steps of
a) determining the level of heat shock protein 27 (HSP27) in a sample of an individual suffering from lung carcinoma,
b) comparing the determined level of HSP27 in said sample with a reference range of HSP27 measured in samples of control individuals with an early stage lung carcinoma and/or with advanced stage lung carcinoma,
c) diagnosing early stage lung carcinoma in said individual when the level of HSP27 in sample a) is below the reference range of HSP27 in samples of control individuals with advanced stage lung carcinoma or within the reference range of control individuals with early stage lung carcinoma and diagnosing advanced stage lung carcinoma when the level of HSP27 in sample a) is above the reference range of HSP27 in samples of control individuals with early stage lung carcinoma or within the reference range of control individuals with advanced stage lung carcinoma.

2. Method according to claim 1, **characterised in that** the early stage lung carcinoma is stage Ia to IIb and advanced stage lung carcinoma is stage IIIa to IV as defined by the International Association for the Study of Lung Cancer (IASCL).

3. Method according to claim 1 or 2, **characterised in that** the sample is a blood sample, preferably a serum or plasma sample.

4. Method according to any one of claims 1 to 3, **characterised in that** the level of HSP27 is determined by an immunoassay, preferably by a competitive or a non-competitive immunoassay, more preferably by a Western-blot, an enzyme-linked immunosorbent assay (ELISA), lateral flow immunoassay or a radioimmunoassay (RIA).

5. Method according to any one of claims 1 to 4, **characterised in that** the reference level of HSP27 in control individuals with an early stage lung carcinoma amounts to 3000 to 4400 pg/ml, preferably 3500 to 4200 pg/ml.

6. Method according to any one of claims 1 to 5, **characterised in that** the reference level of HSP27 in control individuals with an advanced stage lung carcinoma amounts to at least 4600 pg/ml, preferably 4600 to 6000 pg/ml, more preferably 5000 to 5600 pg/ml.

## Patentansprüche

1. Verfahren zum Unterscheiden zwischen einem Lungenkarzinom im Frühstadium und einem Lungenkarzinom im fortgeschrittenen Stadium bei einem Individuum, das an einem Lungenkarzinom leidet, welches die folgenden Schritte umfasst:
a) Feststellen der Konzentration von Hitzeschockprotein 27 (HSP27) in einer Probe von einem Individuum, das an einem Lungenkarzinom leidet,
b) Vergleichen der festgestellten Konzentration von HSP27 in der Probe mit einem Vergleichsbereich von HSP27, der in Proben von Kontrollindividuen mit einem Lungenkarzinom im Frühstadium und/oder mit einem Lungenkarzinom im fortgeschrittenen Stadium gemessen wurde,
c) Diagnostizieren eines Lungenkarzinoms im Frühstadium bei dem Individuum, wenn die Konzentration von HSP27 in Probe a) unter dem Vergleichsbereich von HSP27 in Proben von Kontrollindividuen mit einem Lungenkarzinom im fortgeschrittenen Stadium oder innerhalb des Vergleichsbereichs von Kontrollindividuen mit einem Lungenkarzinom im Frühstadium liegt, und Diagnostizieren eines Lungenkarzinoms im fortgeschrittenen Stadium, wenn die Konzentration von HSP27 in Probe a) über dem Vergleichsbereich von HSP27 in Proben von Kontrollindividuen mit einem Lungenkarzinom im Frühstadium oder innerhalb des Vergleichsbereichs von Kontrollindividuen mit einem Lungenkarzinom im fortgeschrittenen Stadium liegt.

2. Verfahren nach Anspruch 1, **dadurch gekennzeichnet, dass** es sich bei dem Lungenkarzinom im Frühstadium um Stadium Ia bis IIb handelt und es sich bei dem Lungenkarzinom im fortgeschrittenen Stadium um Stadium IIIa bis IV nach der Definition der International Association for the Study of Lung Cancer (IASCL) handelt.

3. Verfahren nach Anspruch 1 oder 2, **dadurch gekennzeichnet, dass** es sich bei der Probe um eine Blutprobe, vorzugsweise um eine Serum- oder Plasmaprobe handelt.

4. Verfahren nach einem der Ansprüche 1 bis 3, **dadurch gekennzeichnet, dass** die Konzentration von HSP27 mit einem Immunassay, vorzugsweise mit einem kompetitiven oder nicht-kompetitiven Immunassay, insbesondere mit einem Western-Blot, einem ELISA (Enyzme-Linked Immunosorbent Assay), einem Immunassay mit Lateralfluss oder einem Radioimmunassay (RIA) festgestellt wird.

5. Verfahren nach einem der Ansprüche 1 bis 4, **dadurch gekennzeichnet, dass** der Vergleichsbereich von HSP27 in Kontrollindividuen mit einem Lungenkarzinom im Frühstadium 3.000 bis 4.400 pg/ml, vorzugsweise 3.500 bis 4.200 pg/ml beträgt.

6. Verfahren nach einem der Ansprüche 1 bis 5, **dadurch gekennzeichnet, dass** der Vergleichsbereich von HSP27 in Kontrollindividuen mit einem Lungenkarzinom im fortgeschrittenen Stadium mindestens 4.600 pg/ml, vorzugsweise 4.600 bis 6.000 pg/ml, insbesondere 5.000 bis 5.600 pg/ml beträgt.

## Revendications

1. Procédé de discrimination entre un carcinome pulmonaire en stade précoce et un carcinome pulmonaire en stade avancé chez un individu souffrant d'un carcinome pulmonaire, ledit procédé comprenant les étapes consistant à :
a) déterminer le taux de protéine de choc thermique 27 (HSP27) dans un échantillon d'un individu souffrant d'un carcinome pulmonaire,
b) comparer le taux déterminé de HSP27 dans ledit échantillon à une plage de référence de HSP27 mesurée dans des échantillons d'individus témoins ayant un carcinome pulmonaire en stade précoce et/ou ayant un carcinome pulmonaire en stade avancé,
c) diagnostiquer un carcinome pulmonaire en stade précoce chez ledit individu lorsque le taux de HSP27 dans l'échantillon a) est au-dessous de la plage de référence de HSP27 dans les échantillons d'individus témoins ayant un carcinome pulmonaire en stade avancé ou dans la plage de référence d'individus témoins ayant un carcinome pulmonaire en stade précoce, et diagnostiquer un carcinome pulmonaire en stade avancé lorsque le taux de HSP27 dans l'échantillon a) est au-dessus de la plage de référence de HSP27 dans les échantillons d'individus témoins ayant un carcinome pulmonaire en stade précoce ou dans la plage de référence d'individus témoins ayant un carcinome pulmonaire en stade avancé.

2. Procédé selon la revendication 1, **caractérisé en ce que** le carcinome pulmonaire en stade précoce est en stade Ia à IIb et le carcinome pulmonaire en stade avancé est en stade IIIa à IV tel que défini par l'Association Internationale pour l'Etude du Cancer du Poumon (IASCL).

3. Procédé selon la revendication 1 ou 2, **caractérisé en ce que** l'échantillon est un échantillon de sang, de préférence un échantillon de sérum ou de plasma.

4. Procédé selon l'une quelconque des revendications 1 à 3, **caractérisé en ce que** le taux de HSP27 est déterminé par un dosage immunologique, de préférence par un dosage immunologique compétitif ou un dosage immunologique non compétitif, de manière davantage préférée par un transfert de type Western, un dosage ELISA (enzyme-linked immunosorbent assay), un dosage immunologique à flux latéral ou un dosage radioimmunologique (RIA).

5. Procédé selon l'une quelconque des revendications 1 à 4, **caractérisé en ce que** le taux de référence de HSP27 chez les individus témoins souffrant d'un carcinome pulmonaire en stade précoce atteint 3 000 à 4 400 pg/mL, de préférence 3 500 à 4 200 pg/mL.

6. Procédé selon l'une quelconque des revendications 1 à 5, **caractérisé en ce que** le taux de référence de HSP27 chez les individus témoins souffrant d'un carcinome pulmonaire en stade avancé atteint au moins 4 600 pg/mL, de préférence de 4 600 à 6 000 pg/mL, de manière davantage préférée de 5 000 à 5 600 pg/mL.
